# EUROPÄISCHE PATENTSCHRIFT

(11) **EP 0 444 011 B1**
(45) Veröffentlichungstag und Bekanntmachung des Hinweises auf die Patenterteilung: **09.06.1993**
(21) Anmeldenummer: 91890026.7
(22) Anmeldetag: 18.02.1991
(51) Int. Cl.: A61M 5/44, A61M 1/02, A61M 1/36

(54) **Vorrichtung zum Erwärmen von Flüssigkeiten, insbesondere zum Erwärmen von Infusions- und/oder Transfusionsflüssigkeiten**
Arrangement for heating liquids, in particular for heating infusion and/or transfusion liquids
Dispositif de chauffage de liquides, notamment de liquides d'infusion et/ou de transfusion

(30) Priorität: 19.02.1990 DE 9001923 U
(43) Veröffentlichungstag der Anmeldung: 28.08.1991
(73) Patentinhaber: ERNST BIEGLER GesmbH, A-3001 Mauerbach (AT)
(72) Erfinder: Biegler, Herbert, 3001 Mauerbach (DE)
(74) Vertreter: Wolfram, Gustav, Dipl.-Ing.

(56) Entgegenhaltungen:
- EP-A- 0 181 447
- DE-A- 3 606 930
- DE-A- 3 726 453

## Beschreibung

Die Erfindung betrifft eine Vorrichtung zum Erwärmen von Flüssigkeiten, insbesondere zum Erwärmen von Infusions- und/oder Transfusionsflüssigkeiten auf eine vorbestimmte Temperatur, mit einem Wärmetauschzylinder, an dessen Oberfläche eine Nut in Schraubenlinienform angeordnet ist, in die eine die Flüssigkeit führende Schlauchleitung einlegbar ist.

Eine Vorrichtung dieser Art ist aus der EP-A-0 181 447 bekannt. Bei dieser Vorrichtung ist die Tiefe der Nut etwas geringer gehalten als der Außendurchmesser der Schlauchleitung, sodaß die Schlauchleitung über die Oberfläche des Wärmetauschzylinders hinausragt.

Zur Vermeidung von Wärmeverlusten ist bei dem bekannten Gerät eine Wärmeschutzmanschette vorgesehen, die den Wärmetauschzylinder außenseitig umgibt und am über die Oberfläche des Wärmetauschzylinders vorstehenden Teil der Schlauchleitung anliegt und so die Schlauchleitung in der Nut hält bzw. in diese preßt.

Eine solche Wärmeschutzmanschette ist insoferne nachteilig, als sie eine eigene Manipulation beim Anbringen der Schlauchleitung erfordert. Zudem stellt die Wärmeschutzmanschette einen eigenen Bestandteil dar, der verloren gehen kann. Bei einem Verlust der Wärmeschutzmanschette ist die Anwärmung der Flüssigkeit auf die vorbestimmte Temperatur nicht mehr gewährleistet. Zudem kann es zu einem Lockern und Herausgleiten der Schlauchleitung aus der Nut kommen, welche Gefahr auch vor dem Auflegen und Fixieren der Wärmeschutzmanschette besteht.

Die Erfindung bezweckt die Vermeidung dieser Nachteile und Schwierigkeiten und stellt sich die Aufgabe, eine Vorrichtung der eingangs beschriebenen Art zu schaffen, bei der ohne Verwendung einer Wärmeschutzmanschette eine rasche und gleichmäßige Erwärmung der Flüssigkeit auf eine vorbestimmte Temperatur unter Verwendung einer möglichst kurzen Schlauchleitung erzielt werden kann und bei der die Schlauchleitung trotzdem zuverlässig an der Vorrichtung fixiert ist. Weiters soll die Anbringung der Schlauchleitung mit unterschiedlichen Windungszahlen am Wärmetauschzylinder in einfacher Weise und ohne zusätzliche Maßnahmen bzw. Hilfsmittel möglich sein.

Diese Aufgabe wird erfindungsgemäß dadurch gelöst, daß die Nut im Querschnitt folgende Merkmale aufweist:
- die Nuttiefe ist größer als die Nutbreite,
- der Nutgrund ist in Richtung der Achse des Wärmetauschzylinders gegenüber der Nutöffnung versetzt angeordnet, wobei
- die den Nutgrund mit der Nutöffnung verbindenden Seitenwände gegenüber der Achse des Wärmetauschzylinders in dieselbe Richtung geneigt sind.

Eine besonders gute Klemmung des Schlauches ist dadurch erzielbar, daß die den Nutgrund mit der Nutöffnung verbindenden Seitenwände eine sich über die gesamte Länge der Nut erstreckende Engstelle aufweisen, deren Breite geringer bemessen ist als die größte Breite des Nutgrundes, wobei der Querschnitt des Nutgrundes dem Außenumriß der Schlauchleitung angepaßt ist.

Eine gute Wärmeisolation nach außen ist dadurch erzielbar, daß die Neigung der den Nutgrund mit der Nutöffnung verbindenden Seitenwände zur Achse des Wärmetauschzylinders mindestens 30°, vorzugsweise 45°, beträgt, wobei vorteilhaft der Nutgrund kreisbogenförmig gestaltet ist und ausgehend von einer Seitenwand der Nut bis zur gegenüberliegenden Seitenwand sich über mindestens 210°, vorzugsweise 225°, erstreckt.

Weiters ist es für eine gleichmäßige Erwärmung der Flüssigkeit von Vorteil, daß die Ganghöhe der Nut geringer ist als die dreifache Breite der Nut an ihrer Engstelle.

Ein einfaches Einlegen der Schlauchleitung wird zweckmäßig dadurch sichergestellt, daß die Seitenwand der Nut, die mit der Oberfläche des Wärmetauschzylinders einen spitzen Winkel einschließt, in die Oberfläche des Wärmetauschzylinders mit einer Abrundung übergeht.

Vorzugsweise ist die Engstelle von den Seitenwänden der Nut gebildet, die zueinander parallel sind.

Die Erfindung ist nachfolgend anhand eines Ausführungsbeispieles näher beschrieben.

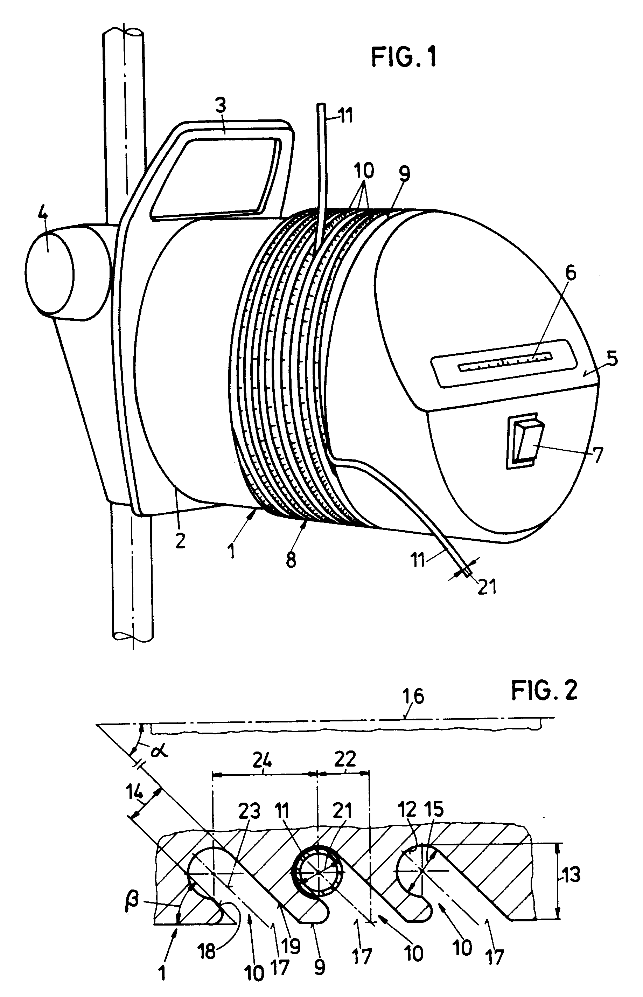

Die Zeichnung zeigt in Fig. 1 die Vorrichtung zum Erwärmen von Flüssigkeiten im Schrägriß; Fig. 2 stellt ein Detail eines in Richtung der Achse des Wärmetauschzylinders geführten Schnittes im vergrößerten Maßstab dar.

Die erfindungsgemäße Vorrichtung weist ein im wesentlichen zylindrisches Gehäuse 1 mit Kreisquerschnitt auf. Dieses Gehäuse 1 ist an einem Ende 2 mit einem Haltegriff 3 und einer Klemme 4 zum Befestigen an einem Traggestell versehen. Am gegenüberliegenden Stirnende 5 ist eine Temperaturanzeige 6 vorgesehen. Hier befindet sich auch ein Netzschalter 7 zum Ein- und Ausschalten des Gerätes. Etwa mittig der Längsausstreckung des Gehäuses 1 ist ein aus Aluminiumguß gefertigter Wärmetauschzylinder 8 eingebaut, der von innen elektrisch beheizbar ist und dessen Temperatur mittels einer Regeleinrichtung, die im Inneren des Gehäuses 1 angeordnet ist, konstant auf einem vorbestimmten Wert gehalten werden kann.

An der kreiszylindrischen Oberfläche 9 des Wärmetauschzylinders 8 ist eine Nut 10 in Schraubenlinienform angeordnet. In diese Nut ist eine die zu erwärmende Flüssigkeit führende Schlauchleitung 11 einleg- bzw. einklemmbar.

Die Nut 10 weist im Querschnitt eine spezifische Gestalt auf (vgl. Fig. 2 ):

Der kreisbogenförmige Nutgrund 12 befindet sich in einer Tiefe 13 unterhalb der Oberfläche 9 des Wärmetauschzylinders 8, die größer ist als die Nutbreite 14. Die Tiefe 13 beträgt etwa das Eineinhalbfache des Durchmessers 15 des kreisbogenförmigen Nutgrundes 12.

Weiters ist der Nutgrund 12 in Richtung der Achse 16 des Wärmetauschzylinders 8 gegenüber der Nutöffnung 17 versetzt angeordnet, wobei die den Nutgrund 12 mit der Nutöffnung 17 verbindenden Seitenwände 18, 19 der Nut 10 gegenüber der Achse 16 des Wärmetauschzylinders 8 in dieselbe Richtung geneigt sind. Diese Wände 18, 19 sind beim dargestellten Ausführungsbeispiel zueinander parallel ausgerichtet.

Die der Nutbreite 14 entsprechende Distanz der Seitenwände der Nut - im rechten Winkel zu den Seitenwänden 18, 19 gemessen - ist etwas geringer als der Durchmesser 15 des kreisbogenförmigen Nutgrundes 12. Hiedurch wird eine sich über die gesamte Länge der Nut 10 erstreckende Engstelle gebildet. Die Neigung alpha der Seitenwände 18, 19 zur Achse 16 des Wärmetauschzylinders 8 beträgt etwa 45°.

Der Durchmesser 15 des Nutgrundes 12 entspricht dem Durchmesser 21 der einzulegenden Schlauchleitung 11. Infolge der Verengung wird die Schlauchleitung 11 über einen Winkel (in Querschnittsrichtung gemessen) von etwa 225° umhüllt. Die Übergänge der Nutseitenwände 18,19 zur Oberfläche 9 des Wärmetauschzylinders 8 sind abgerundet ausgebildet, um die Schlauchleitung 11 beim Einlegen in die Nut 10 nicht zu verletzen.

Durch die Versetzung 22 des Nutgrundes 12 gegenüber der Nutöffnung 17, also die schräge Querschnitts-Mittelachse 23 der Nut 10, ist eine weitgehende Wärmeabschirmung der Schlauchleitung 11 sichergestellt, sodaß eine Wärmeschutzmanschette nicht erforderlich ist.

Die große Nuttiefe 13 ermöglicht infolge der hiedurch bewirkten Höhe der Seitenwände 18,19 eine einwandfreie Klemmung der Schlauchleitung 11, sodaß ein Festhalten der Schlauchleitung 11 mittels einer Manschette oder mittels eines Haltebügels nicht erforderlich ist.

Zweckmäßig ist die Ganghöhe 24 der Nut 10 geringer als die dreifache Breite 14 der Nut 10 an ihrer Engstelle. Durch diese eng benachbarte Anordnung der Windungen des Schlauches erfolgt eine optimale Ausnützung der eingebrachten Wärmeenergie und läßt sich eine hohe Temperaturkonstanz erzielen.

Das Einlegen der Schlauchleitung 11 kann in einfacher Weise durch Ausübung einer Zugkraft in Längsrichtung der Schlauchleitung 11 bei gleichzeitigem Winden der Schlauchleitung 11 um den Wärmetauschzylinder 8 bewirkt werden. Dabei gleitet die Schlauchleitung 11 bis zum Nutgrund 12. Eine darüber hinausgehende Manipulation ist nicht erforderlich. Zum Entnehmen der Schlauchleitung 11 aus der Nut 10 ist lediglich eine Zugkraft, die etwa quer zur Nut 10 gerichtet ist, notwendig.

## Patentansprüche

1. Vorrichtung zum Erwärmen von Flüssigkeiten, insbesondere zum Erwärmen von Infusions- und/oder Transfusionsflüssigkeiten auf eine vorbestimmte Temperatur, mit einem Wärmetauschzylinder (8), an dessen Oberfläche (9) eine Nut (10) in Schraubenlinienform angeordnet ist, in die eine die Flüssigkeit führende Schlauchleitung (11) einlegbar ist, dadurch gekennzeichnet, daß die Nut (10) im Querschnitt folgende Merkmale aufweist:
- daß die Nuttiefe (13) größer ist als die Nutbreite (14),
- daß der Nutgrund (12) in Richtung der Achse (16) des Wärmetauschzylinders (8) gegenüber der Nutöffnung (17) versetzt angeordnet ist, wobei
- die den Nutgrund (12) mit der Nutöffnung (17) verbindenden Seitenwände (18, 19) gegenüber der Achse (16) des Wärmetauschzylinders (8) in dieselbe Richtung geneigt sind.

2. Vorrichtung nach Anspruch 1, dadurch gekennzeichnet, daß die den Nutgrund (12) mit der Nutöffnung (17) verbindenden Seitenwände (18, 19) eine sich über die gesamte Länge der Nut (10) erstreckende Engstelle bilden, deren Breite (14) geringer bemessen ist als die größte Breite (15) des Nutgrundes (12), wobei der Querschnitt des Nutgrundes (12) dem Außenumriß der Schlauchleitung (11) angepaßt ist.

3. Vorrichtung nach Anspruch 1 oder 2, dadurch gekennzeichnet, daß die Neigung alpha der den Nutgrund (12) mit der Nutöffnung (17) verbindenden Seitenwände (18, 19) zur Achse (16) des Wärmetauschzylinders (8) mindestens 30°, vorzugsweise 45°, beträgt.

4. Vorrichtung nach Anspruch 3, dadurch gekennzeichnet, daß der Nutgrund (12) kreisbogenförmig gestaltet ist und ausgehend von einer Seitenwand (18) der Nut (10) bis zur gegenüberliegenden Seitenwand (19) sich über mindestens 210°, vorzugsweise 225°, erstreckt.

5. Vorrichtung nach einem oder mehreren der Ansprüche 2 bis 4, dadurch gekennzeichnet, daß die Ganghöhe (24) der Nut (10) geringer ist als die dreifache Breite (14) der Nut (10) an ihrer Engstelle.

6. Vorrichtung nach einem oder mehreren der Ansprüche 1 bis 5, dadurch gekennzeichnet, daß die Seitenwand (18) der Nut (10), die mit der Oberfläche (9) des Wärmetauschzylinders (8) einen spitzen Winkel beta einschließt, in die Oberfläche (9) des Wärmetauschzylinders (8) mit einer Abrundung übergeht.

7. Vorrichtung nach einem oder mehreren der Ansprüche 2 bis 6, dadurch gekennzeichnet, daß die Engstelle von den Seitenwänden (18, 19) der Nut (10) gebildet ist, die zueinander parallel sind.

## Claims

1. An arrangement for heating liquids, in particular for heating infusion and/or transfusion liquids to a predetermined temperature, comprising a heat exchange cylinder (8) on whose surface (9) a helical groove (10) is provided, into which a hose duct (11) conducting the liquid is insertable, characterized in that the groove (10) has the following characteristic features in terms of cross section:
- the depth (13) of the groove is larger than the width (14) of the groove,
- the bottom (12) of the groove is arranged to be offset relative to the opening (17) of the groove in the direction of the axis (16) of the heat exchange cylinder (8), wherein
- the side walls (18, 19) connecting the bottom (12) of the groove with the opening (17) of the groove are inclined relative to the axis (16) of the heat exchange cylinder (8) in the same direction.

2. An arrangement according to claim 1, characterized in that the side walls (18, 19) connecting the bottom (12) of the groove with the opening (17) of the groove constitute a narrow extending over the total length of the groove (10) and whose width (14) is dimensioned to be smaller than the largest width (15) of the bottom (12) of the groove, the cross section of the bottom (12) of the groove being adapted to the outer contour of the tube duct (11).

3. An arrangement according to claim 1 or 2, characterized in that the inclination alpha of the side walls (18, 19) connecting the bottom (12) of the groove with the opening (17) of the groove, relative to the axis (16) of the heat exchange cylinder (8) amounts to at least 30°, preferably to 45°.

4. An arrangement according to claim 3, characterized in that the bottom (12) of the groove is circularly-arc-shaped, extending from a side wall (18) of the groove (10) as far as to the opposite side wall (19) over at least 210°, preferably over 225°.

5. An arrangement according to one or several of claims 2 to 4, characterized in that the pitch (24) of the groove (19) is smaller than three times the width (14) of the groove (10) at its narrow.

6. An arrangement according to one or several of claims 1 to 5, characterized in that the side wall (18) of the groove (10), which encloses an acute angle beta with the surface (9) of the heat exchange cylinder (8), merges into the surface (9) of the heat exchange cylinder (8) by a rounding.

7. An arrangement according to one or several of claims 2 to 6, characterized in that the narrow is formed by the side walls (18, 19) of the groove (10), that are parallel to each other.

## Revendications

1. Dispositif pour chauffer des liquides, en particulier pour chauffer des liquides de perfusion et/ou de transfusion à une température prédéterminée, comportant un cylindre échangeur de chaleur (8) sur la surface (9) duquel est prévue une rainure (10) en forme d'hélice dans laquelle peut être insérée une conduite en tuyau souple (11) qui véhicule le liquide, caractérisé en ce que la rainure (10) présente en section droite les caractéristiques suivantes :
- la profondeur (13) de la rainure est supérieure à la largeur (14) de la rainure,
- le fond (12) de la rainure est décalé par rapport à l'ouverture (17) de la rainure dans la direction de l'axe (16) du cylindre échangeur de chaleur (8),
- les parois latérales (18, 19) qui relient le fond (12) de la rainure à l'ouverture (17) de la rainure étant inclinées dans la même direction par rapport à l'axe (16) du cylindre échangeur de chaleur (8).

2. Dispositif selon la revendication 1, caractérisé en ce que les parois latérales (18, 19) qui relient le fond (12) de la rainure à l'ouverture (17) de la rainure forment un rétrécissement qui s'étend sur toute la longueur de la rainure (10) et dont la largeur (14) est de plus faible dimension que la plus grande largeur (15) du fond (12) de la rainure, la section droite du fond (12) de la rainure étant adaptée au contour externe de la conduite en tuyau souple (11).

3. Dispositif selon la revendication 1 ou 2, caractérisé en ce que l'inclinaison α des parois latérales (18, 19) qui relient le fond (12) de la rainure à l'ouverture (17) de la rainure par rapport à l'axe (16) du cylindre échangeur de chaleur (8) est d'au moins 30°, de préférence de 45°.

4. Dispositif selon la revendication 3, caractérisé en ce que le fond (12) de la rainure est en forme d'arc de cercle et s'étend depuis une paroi latérale (18) de la rainure (10) jusqu'à la paroi latérale (19) opposée sur au moins 210°, de préférence sur 225°.

5. Dispositif selon une ou plusieurs des revendications 2 à 4, caractérisé en ce que le pas (24) de la rainure (10) est plus petit que le triple de la largeur (14) de la rainure (10) au niveau de son rétrécissement.

6. Dispositif selon une ou plusieurs des revendications 1 à 5, caractérisé en ce que la paroi latérale (18) de la rainure (10), qui forme avec la surface (9) du cylindre échangeur de chaleur (8) un angle aigu β, forme une transition arrondie avec la surface (9) du cylindre échangeur de chaleur (8).

7. Dispositif selon une ou plusieurs des revendications 2 à 6, caractérisé en ce que le rétrécissement est formé par les parois latérales (18, 19) de la rainure (10) qui sont parallèles entre elles.
